# EUROPEAN PATENT SPECIFICATION

(11) **EP 1 698 637 B1**
(45) Date of publication and mention of the grant of the patent: **15.10.2008**
(21) Application number: 06003720.7
(22) Date of filing: 23.02.2006
(51) Int. Cl.: C07K 1/16, C07K 1/34

(54) **A method for improving recovery yield in protein purification with gel filtration chromatography using arginine**
Methode zur Verbesserung der Ausbeute bei der Proteinreinigung mittels Gelfiltrationschromatographie und Arginin
Méthode pour améliorer la récupération d'une protéine en moyens de filtration sur gel chromatographie et arginine

(30) Priority: 03.03.2005 US 657735 P
(43) Date of publication of application: 06.09.2006
(73) Proprietor: Ajinomoto Co., Inc., Tokyo (JP)
(72) Inventor: Ejima, Daisuke c/o Ajinomoto Co., Inc., Kawasaki-shi Kanagawa, 210-8681 (JP); Yumioka, Ryosuke c/o Ajinomoto Co., Inc., Kawasaki-shi Kanagawa, 210-8681 (JP); Arakawa, Tsutomu, Thousand Oaks, CA 91360 (US)
(74) Representative: Strehl Schübel-Hopf & Partner

(56) References cited:
- EP-A- 1 568 710
- TSUMOTO KOUHEI ET AL: "Role of arginine in protein refolding, solubilization, and purification" BIOTECHNOLOGY PROGRESS, vol. 20, no. 5, September 2004 (2004-09), pages 1301-1308, XP002383385 ISSN: 8756-7938
- UMETSU M ET AL: "Nondenaturing solubilization of beta2 microglobulin from inclusion bodies by l-arginine" BIOCHEMICAL AND BIOPHYSICAL RESEARCH COMMUNICATIONS, ACADEMIC PRESS INC. ORLANDO, FL, US, vol. 328, no. 1, 6 January 2005 (2005-01-06), pages 189-197, XP004723758 ISSN: 0006-291X
- STULIK KAREL ET AL: "Some potentialities and drawbacks of contemporary size-exclusion chromatography." JOURNAL OF BIOCHEMICAL AND BIOPHYSICAL METHODS, vol. 56, no. 1-3, 30 June 2003 (2003-06-30), pages 1-13, XP002383386 ISSN: 0165-022X
- BLEEKER WIM K ET AL: "Vasoactive side effects of intravenous immunoglobulin preparations in a rat model and their treatment with recombinant platelet-activating factor acetylhydrolase" BLOOD, vol. 95, no. 5, 1 March 2000 (2000-03-01), pages 1856-1861, XP002383387 ISSN: 0006-4971
- ISHIBASHI M ET AL: "Is arginine a protein-denaturant?" PROTEIN EXPRESSION AND PURIFICATION, ACADEMIC PRESS, SAN DIEGO, CA, US, vol. 42, no. 1, 19 April 2005 (2005-04-19), pages 1-6, XP004918440 ISSN: 1046-5928

## Description

### [Technical Field]

The present invention relates to a method for improving recovery yield of a protein that is hard to be recovered in a peak with gel filtration chromatography using a water soluble buffer, thereby enabling a quantitative analysis and a preparative operation. More specifically, the invention relates to the recovery enhancing substance which is added to an developing solvent for use in improving recovery yields of antibodies, hydrophobic proteins or hydrophobic peptides.

### [Background Art]

Knowledge of molecular weight distribution of a protein in a solution is an extremely important factor in defining a molecular association unit required for expressing the function of the protein. Further, extremely significant problems are raised by the states of association of proteins which are different from the natural state to permit expression of the function thereof; or the state of exerting a distinct action from the expected action (i.e., adverse effect), or losing its stability. For example, monoclonal antibodies for use in therapy exert proper action and stability as long as they are kept in the state of a monomer, however, it is known that when multiple molecules associate to form aggregates, serious adverse effect as well as antigenicity, deterioration of storage stability may be caused [Nonpatent Document 1, 2]. Examination of molecular weight distribution of a protein is regarded as an important technique not only for a basic research of biochemistry, but also for research and development as well as quality control of proteinous pharmaceutical products. Examples of known method of the examination of molecular weight distribution of a protein include gel filtration chromatography, ultracentrifugal analysis, electrophoretic analysis, light scattering analysis, dynamic light scattering analysis and the like. Recently, X-ray small angle scattering has also been utilized as a practical technique. However, among them, gel filtration chromatography is a technique that is most frequently used entirely in from laboratories of proteins over production fields, as a method which can determine the protein molecular weight distribution in an aqueous solution in a most simple manner. Gel filtration chromatography is referred to as capable of providing the information that is comparable to ultracentrifugal analysis by using not only ultraviolet absorption but also light scattering, refractive index, density in combination as a detection process [Nonpatent Document 3]. However, there still remain problems to be dissolved in gel filtration chromatography. Proteins follow the elution order depending on their molecular weight through diffusion in voids of the stationary phase provided by the column in gel filtration chromatography. This elution order in terms of each retention time is converted into the molecular weight information, however, the protein not only distributes in the voids of the stationary phase, but also interacts with the stationary phase itself. When the interaction is potent, the elution order may be retarded and may possibly affect the molecular weight information. Because interaction of the aggregates of the protein with the filler is particularly strong, elution from the column in a peak may also be completely inhibited. In such instances, despite of inclusion of the aggregates, completely erroneous conclusion that any aggregates is not included at all may be drawn by the analysis with gel filtration chromatography. It has been generally comprehended that gel filtration chromatography of not alone the aggregates but highly hydrophobic proteins or peptides, in general, is difficult. Taking into account of the foregoing current circumstances, a proposal was made to perform gel filtration chromatography in a more appropriate manner by optimizing combination of the stationary phase, the mobile phase (developing solvent) and detection method [Nonpatent Document 4], however, any advantageous procedure enabling alleviation of interaction between a protein aggregates, a highly hydrophobic protein or a highly hydrophobic peptide and a column stationary phase, to perfect the recovery in a peak has not been found. As reported by Stulik et al. [Nonpatent Document 4], even though an additive which is expected to alleviate the interaction between a column stationary phase and a protein such as e.g., an acid, an inorganic salt or an organic solvent is added to the mobile phase in an appropriate amount, successive improvement of separation of the aggregates is exceptionally achieved. Further, addition of a protein denaturating agent, which is expected to efficaciously alleviate the interaction, into the mobile phase just in an appropriate amount was also reported, however, difficulty in setting of the condition shall be obvious due to the risk of concomitantly evoked protein degeneration. Accordingly, in performing gel filtration chromatography of a protein including aggregates, a highly hydrophobic protein or a highly hydrophobic peptide, persons in the art have strongly desired to find a condition for quantitatively recovering a peak of the aggregates or the hydrophobic protein without enormous studies on the condition.
[Nonpatent Document 1] Pharmaceutical Research, 11 (1994), 764-771
[Nonpatent Document 2] Blood, 95 (2000), 1856-1861
[Nonpatent Document 3] Analytical Biochemistry, 325 (2004), 227-239
[Nonpatent Document 4] Journal of Biochemical and Biophysical Methods, 56 (2003), 1-13

### [Disclosure of the Invention]

### [Problems that the Invention is to Solve]

An object of the present invention is to more quantitatively recover a peak of a protein aggregates, hydrophobic proteins or hydrophobic peptides, which are conventionally difficult to be recovered and detected as a peak, using a commercially available gel filtration chromatography column and a mobile phase with a little study on the condition, and more specifically, to find a compound which may be added to and has not been conventionally used in a mobile phase.

### [Means for Solving the Problems]

Accordingly, an aspect of the present invention is to provide a method for more quantitatively recovering aggregates, a hydrophobic protein or a hydrophobic peptide in performing gel filtration chromatography of proteins including the aggregates, the hydrophobic proteins or the hydrophobic peptides, through adding an appropriate amount of arginine into a water soluble buffer of the developing solvent which is used for each, thereby attenuating unnecessary interaction that occurs between the proteins and the column stationary phase.

### [Advantage of the Invention]

Interaction caused between the column stationary phase and the protein, which involved a significant problem conventionally, can be alleviated by merely adding arginine into a water soluble buffer of the developing solvent. It has been already known that arginine does not affect the stability or structure of a protein. Therefore, according to the present invention, recovery of a peak in gel filtration chromatography of protein aggregates, hydrophobic proteins or hydrophobic peptides is improved without affecting the structure or stability of proteins. This means that the invention is applicable to not only analyses of proteins or peptides, but also a method for producing proteins in which gel filtration chromatography is employed.

### [Best Mode for Carrying Out the Invention]

The agent for alleviating interaction between protein aggregates, hydrophobic proteins or hydrophobic peptides and a column of gel filtration chromatography which may be used in the present invention is arginine that is a kind of natural amino acid and/or an arginine derivative. Examples of the arginine derivative include, in addition to the arginine, acylated arginine such as acetyl arginine and N-butyloyl arginine, arginine butyl esters with the carboxyl group modified, agmatine with the carboxyl group eliminated, arginine having a hydroxyl group introduced in place of an α-amino group, and the like. These may be used in the form of an acid addition salt. Examples of the acid which can form an acid addition salt include hydrochloric acid and the like.

Furthermore, the buffer as the developing solvent into which the arginine and/or the arginine derivative is added which may be used in the invention may be adjusted to have the pH and buffer concentration in accordance with the property of the target protein aggregates, hydrophobic proteins or hydrophobic peptides, but any particular pH or buffer concentration is not required. Adjustment of the pH may be generally carried out using the buffer in use (for example, sodium phosphate buffer), with no need of adjustment of the pH for the arginine and/or the arginine derivative. The arginine and/or the arginine derivative added to the buffer of the developing solvent may be added to give the concentration of 0.05 to 1.50 M, preferably 0.05 to 1.25 M, and more preferably 0.10 to 0.75 M, whereby the recovery yield of the protein peak can be improved. The arginine and/or the arginine derivative may or may not be added into the solute, i.e., the solution containing an antibody including aggregates, hydrophobic proteins, or hydrophobic peptides. When enhancement of ionic strength of the solution including the solute is intended, the arginine and/or the arginine derivative may be added similarly to other salt.

Next, it is preferred that the elution method which may be used in the invention is an isocratic elution method, generally referred to, in which one kind of buffer is used as the developing solvent.

Moreover, the gel filtration chromatography column used in the invention may be a commercially available product, and examples thereof include e.g., Superdex 200HR 10/30, Superdex 75HR 10/30 (both manufactured by Amersham Bioscience K.K.), TSK G3000SWXL (manufactured by Tosoh Corporation) and the like.

Examples of the protein whose recovery yield of the aggregates or the like can be improved which may be used in the invention include natural human antibodies, or humanized antibodies or human-type antibodies prepared by recombinant DNA techniques, monoclonal antibodies of mouse and the like including aggregates. Irrespective of species and subclass of the antibody, any one is applicable as long as it is an antibody including aggregates.

In connection with the antibody including aggregates, it has been known that an antibody forms so called aggregates through association among molecules during the production step (concentration, exposure to acidic pH, heating manipulation) or storage step (solution, frozen solution, freeze-drying), which is referred to as involving in deterioration of the action and exertion of the adverse effect [Nonpatent Document 5]. Quantitative determination, and separation and removal of the aggregates are extremely important in industrial applications. Although mechanism of formation of the aggregate is not uniform, it can not be readily dissociated into the monomer once formed. It is known that the aggregate exhibits greater hydrophobicity than the monomer, leading to lowered recovery yield on the chromatography.

Furthermore, the invention is also applicable to proteins other than antibodies, which may be proteins or peptides having high hydrophobicity, and involving difficulty in recovering in a peak with use of a water soluble buffer alone. The invention is applicable to any method despite of the method of preparation i.e., extraction from nature, preparation with recombinant DNA techniques or the like.

In connection with the hydrophobic protein and peptide herein, no distinct definition is present, in general, which discriminates proteins and peptides. Although ones having constituting amino acids of 50 or more may be considered to be a protein, this categorization is not generally adopted. According to the invention, soluble compounds which are generally considered to be a peptide or a protein are intended.

Also, the hydrophobic protein and the hydrophobic peptide can not be clearly defined, in general, however, they mean proteins and peptides having high content of hydrophobic amino acids in their constituting amino acids, according to the invention. When proportion of the hydrophobic amino acid is elevated, interaction resulting from the hydrophobic interaction with the chromatography column stationary phase is enhanced. Thus, prolonged elution time is found [Nonpatent Document 6]. The invention is responsible for ameliorating lowering of the recovery yield which results from the hydrophobic interaction in chromatography.

In cases of analysis of an antibody containing 60% aggregates by gel filtration chromatography, use of phosphate buffer alone in the developing solvent often results in recovery yield of the antibody peak being only less than 20% of the amount of the antibody subjected to the column even though whole of the detected peaks are added to give the summation. Further, in this instance, the content of the aggregates is often construed erroneously as being approximately 20% that is greatly below the proper value of 60%. However, when a phosphate buffer to which arginine in an amount of 0.2 M is added is used as the developing solvent, recovered antibody peak reaches almost 100% of the amount of the antibody loaded on the column, without altering any other condition, indicating correct value of 60% for the content of the aggregates. The invention will be specifically explained with reference to Examples below, however, the invention is not anyhow limited to these Examples.
[Nonpatent Document 5] Monoclonal Antibodies, Principles and Applications., p. 231-265, London: Wiley Liss, Inc., 1995
[Nonpatent Document 6] Protein Biotechnology, pp. 67, BAIFUKAN CO., LTD, 1996

### [Example 1]

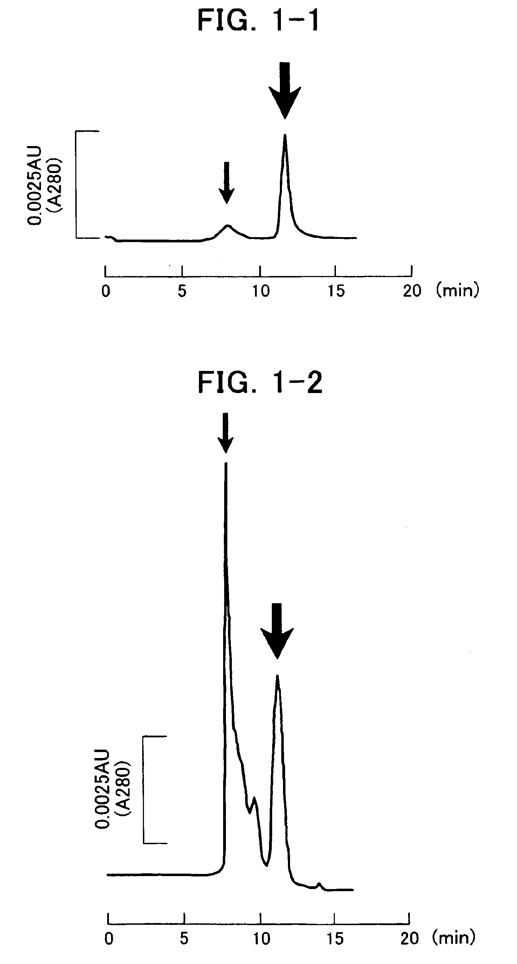

To 120 µl of a 5.28 mg/ml purified anti-von Willebrand factor monoclonal antibody (mouse monoclonal antibody, subclass: IgG₁; WO96/17078) dissolved in an isotonic sodium phosphate buffer was added 30 µl of 0.5 M sodium citrate, pH 2.72 to adjust the pH of 3.04. Thereto were added 60 µl of 0.2 M citric acid, pH 4.50, and further 240 µl of 3 M arginine hydrochloride, pH 4.52, respectively, to adjust the pH of 4.5. This solution was heated at 45°C for 20 min, and thereafter, 67.5 µl of 1 M Tris hydrochloride, pH 8.50 was added thereto to adjust the volume of 517.5 µl, and the pH of 5.13, followed by final heating at 45°C for 10 min. Immediately thereafter, the mixture was cooled at room temperature, and then, concentration of the antibody remained in the supernatant was measured using a spectrophotometer. The antibody concentration was calculated based on the absorbance of 1.4 at a wavelength of 280 nm to be 1 mg/ml. This mouse monoclonal antibody sample after the heat treatment in a volume of 15 µl was subjected to gel filtration chromatography with a column of TSK G3000SWXL using 0.1 M sodium phosphate, pH 6.8 as the developing solvent at a flow rate of 0.8 ml/min, and elution performance of the peak was studied using the ultraviolet absorption at a wavelength of 280 nm as a marker. As shown in Fig. 1-1, a peak of the main component was found at a position that agrees with the elution position of the antibody monomer, while the aggregates was found as a subcomponent peak. A mouse monoclonal antibody in an amount of 5.28 µg which does not include the associated form at all was previously subjected to the same gel filtration chromatography, and the value of peak area per unit weight was determined from the value of area of the antibody peak detected at a wavelength of 280 nm. Using this value, amount of the antibody recovered in a peak was calculated. The results are presented in Table 1. Measurement of the absorbance revealed that the antibody remaining in the supernatant after the heating was 92.4% of that before the heating, however, it was elucidated that total recovery yield of the peaks of the antibody monomer and the aggregates as detected on the gel filtration chromatography was low which accounts for 18.5% of that before the heating, suggesting possible detection of only 1/5 (fifth) of the antibody remained in the supernatant. The content of the aggregates occupying the entire peak then was 21.7%.

Next, 15 µl of the same mouse monoclonal antibody sample after the heat treatment was subjected to gel filtration chromatography in which the completely same condition was employed except that arginine hydrochloride was added to the developing solvent in the aforementioned gel filtration chromatography to give 0.2 M. Thus, as shown in Fig. 1-2, both kind and amount of the detected peaks drastically increased in comparison with the case in which arginine was not added to the developing solvent (Fig. 1-1), exhibiting total recovery yield of the peaks of the detected antibody monomer and the aggregates which was 92.8% of that before heating, which was almost completely consistent with 92.4% that is the recovery yield of the antibody in the supernatant calculated based on the absorbance. The content of the aggregates occupying the entire peaks then was 67.4%. Approximate consistence of the recovery yield of the entire peaks with the value of quantitative determination according to the optical density method suggested that addition of arginine to the buffer of the developing solvent enables quantitative recovery of the aggregates of the antibody, which could not be correctly detected under the conventional condition, and that, as a result, content of the aggregates in the supernatant could be accurately determined.

To the contrary, the content of the aggregates in the case where arginine was not added to the developing solvent was estimated to be as low as 21.7%. This is a quantitative value erroneously obtained due to insufficient recovery yield of the aggregates from the column.

**Table 1 Results of analysis of mouse monoclonal antibody after heat treatment**

| Analysis method | Content of aggregates | Total recovery yield of monomer and associated form⁽*⁾ |
|---|---|---|
| Gel filtration chromatography (Fig. 1-1: sodium phosphate alone) | 21.7% | 18.5% |
| Gel filtration chromatography (Fig. 1-2: sodium phosphate + 0.2 M arginine hydrochloride) | 67.4% | 92.8% |
| Optical density method | -⁽**⁾ | 92.4% |

| | | |
|---|---|---|
| ⁽*⁾ Recovery yield of the amount of the antibody after the heat treatment to the amount of the antibody before the heat treatment ⁽**⁾Antibody monomer and antibody aggregates being indistinguishable | | |

From the results described above, it was proven that when an antibody solution containing aggregates is subjected to gel filtration chromatography to perform purification, the method is efficacious not only for an analysis but also as a method of the production of an antibody because purification by gel filtration chromatography can be performed repeatedly without allowing the aggregates to remain in the column.

### [Example 2]

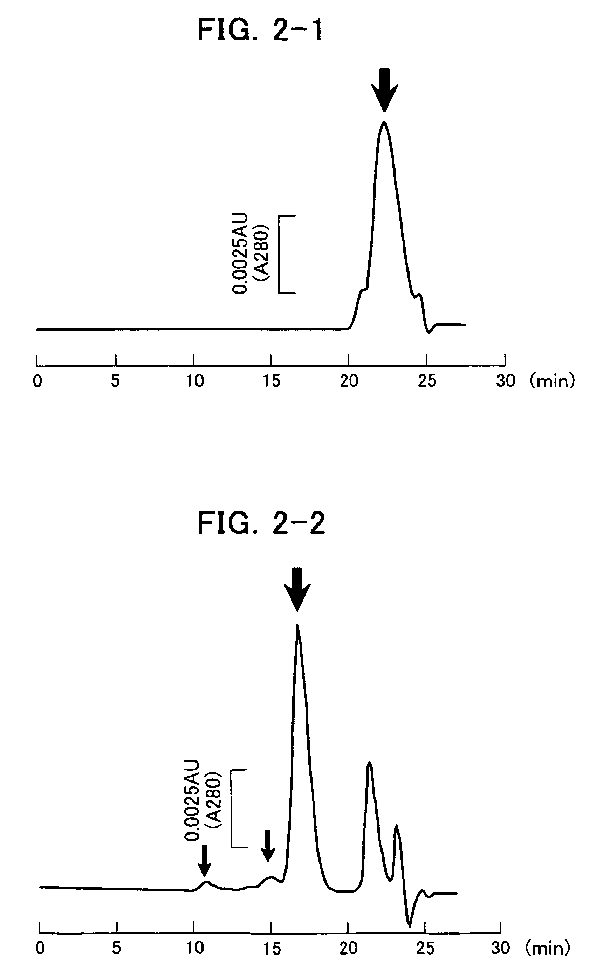

Ten microliter of a solution of 0.70 mg/ml human activin (US Patent Nos. US6084076, and US6756482) dissolved in 70 mM sodium acetate, pH 5.0 was subjected to gel filtration chromatography with a column of Superdex 75HR 10/30 (manufactured by Amersham Bioscience K.K.) using 0.1 M sodium phosphate and 0.75 M sodium chloride, pH 7.3 as the developing solvent at a flow rate of 0.8 ml/min, and elution performance of the peak was studied using ultraviolet absorption at a wavelength of 280 nm as a marker. As shown in Fig. 2-1, human activin was detected at the same position as salts, and the peak area was calculated to be 200290 from the absorption at a wavelength of 280 nm. Any peak was not detected at all at an elution position that was consistent with the molecular weight of human activin (26000 Dalton). The foregoing results suggest that gel filtration chromatography of human activin was not successfully executed, and thus, the content of the aggregates could not be determined. On the other hand, 10 µl of the same solution of human activin was subjected to gel filtration chromatography with the completely same condition except that 0.75 M arginine hydrochloride was added in place of 0.75 M sodium chloride in the developing solvent for the aforementioned gel filtration chromatography. Accordingly, as shown in Fig. 2-2, human activin was found as a symmetric peak at an elution position that was consistent with its molecular weight (26000 Da). The peak area was calculated to be 155027 based on the absorption at a wavelength of 280 nm. Furthermore, a peak of the aggregates was found forward of the human activin, with the peak area of 3687. According to the ratio of the peak area, the content of the aggregates was calculated to be 2.3 %. As described hereinabove, gel filtration chromatography of human activin, the peak of which can be neither separated nor recovered by gel filtration chromatography in which a general buffer is used as the developing solvent, was enabled by merely adding arginine into the developing solvent.

### [Example 3]

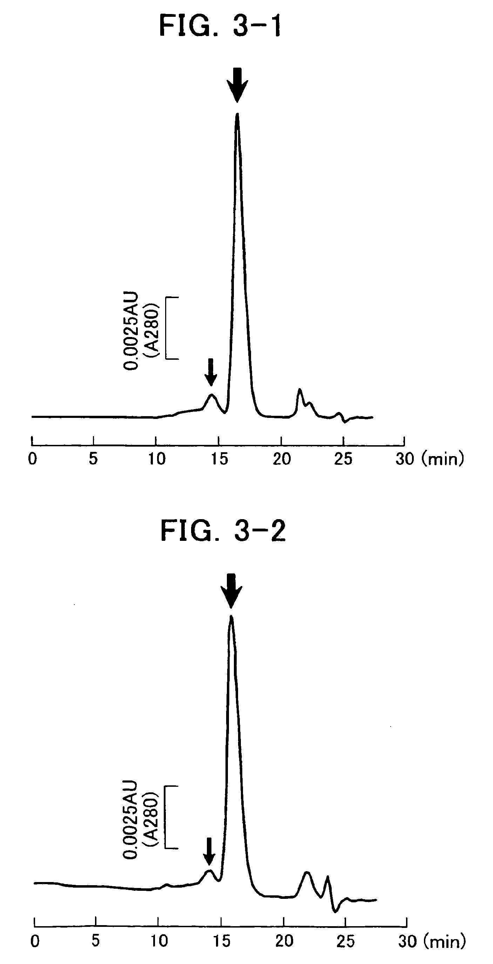

Human interleukin 6 (US Patent No. 5610284) dissolved to give 2.13 mg/ml in 10 mM sodium citrate and 8.7 mM sodium phosphate, pH 7.0 in a volume of 3.5 µl was subjected to gel filtration chromatography with a column of Superdex 75HR 10/30 (manufactured by Amersham Bioscience K.K.) using 0.1 M sodium phosphate, 0.75 M sodium chloride, pH 7.3 as the developing solvent at a flow rate of 0.8 ml/min, and elution performance of the peak was studied using the ultraviolet absorption at a wavelength of 280 nm as a marker. As shown in Fig. 3-1, the monomer and the aggregates of human interleukin 6 were separated. The monomer was detected as a highly symmetric peak at an elution position that was consistent with the molecular weight (21000 Dalton) of human interleukin 6, the peak area of which was calculated to be 182028 based on the absorption at a wavelength of 280 nm. The peak area of the aggregates eluted forward of the monomer was calculated to be 9012. Based on the ratio of the peak area, the content of the aggregates was calculated to be 4.7%. On the other hand, 3.5 µl of the same solution of human interleukin 6 was subjected to gel filtration chromatography with the completely same condition except that 0.75 M arginine hydrochloride was added in place of 0.75 M sodium chloride in the developing solvent for the aforementioned gel filtration chromatography. Accordingly, as shown in Fig. 3-2, human interleukin 6 was found as a symmetric peak at an elution position that was consistent with its molecular weight (21000). The peak area was calculated to be 182226 based on the absorption at a wavelength of 280 nm. Furthermore, the peak area of the aggregates eluted forward of the monomer was calculated to be 4779. According to the ratio of the peak area, the composition ratio of the aggregates was calculated to be 2.6%. It was revealed that human interleukin 6 is favorably separated and recovered in peaks of the monomer and the aggregates in gel filtration chromatography, irrespective of whether the salt added to the developing solvent is sodium chloride or arginine hydrochloride. Also in cases where arginine hydrochloride was added, the aggregates was properly separated and recovered, therefore, it was revealed that arginine hydrochloride in the developing solvent never allows the aggregates to be dissociated into the monomer during progress of the analysis, thereby avoiding presenting unreasonably low content of the aggregates.

### [Example 4]

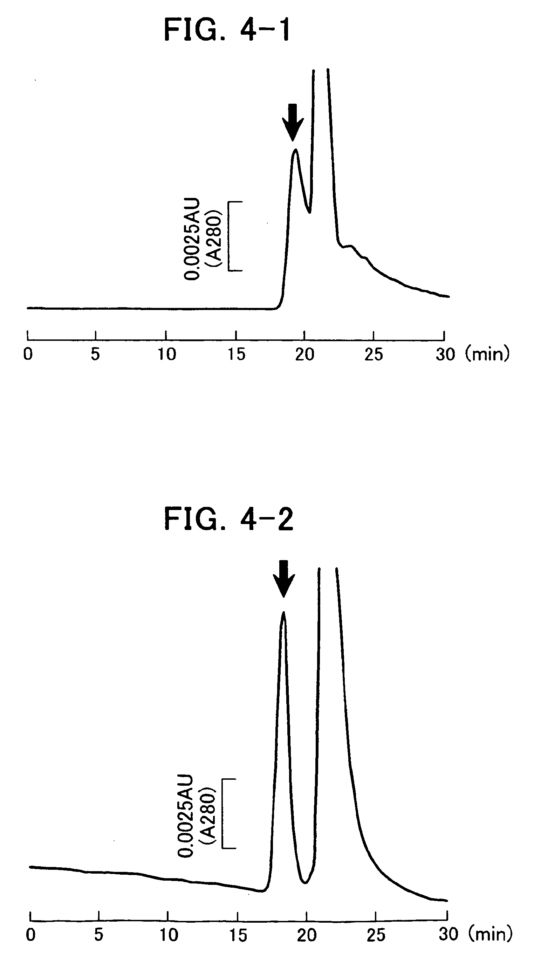

Human fibroblast growth factor (manufactured by Biosource Inc., Catalog No. PHG0026) was dissolved in pure water to adjust to give a concentration of 0.25 mg/ml. This solution in a volume of 20 µl was subjected to gel filtration chromatography with a column of Superdex 75HR 10/30 (manufactured by Amersham Bioscience K.K.) using 0.1 M sodium phosphate, 0.2 M sodium chloride, pH 6.8 as the developing solvent at a flow rate of 0.8 ml/min, and the elution performance of the peak was studied using the ultraviolet absorption at a wavelength of 280 nm as a marker. As shown in Fig. 4-1, peak of the human fibroblast growth factor was found later than the elution position expected from its molecular weight (17000), and the peak area thereof was calculated to be 59281 based on the absorption at a wavelength of 280 nm. On the other hand, 20 µl of the completely same solution of human fibroblast growth factor was subjected to gel filtration chromatography with the completely same condition except that 0.2 M arginine hydrochloride was added in place of 0.2 M sodium chloride in the developing solvent for the aforementioned gel filtration chromatography. Accordingly, as shown in Fig. 4-2, human fibroblast growth factor was eluted at a position slightly forward of that in case where sodium chloride was added as described above, and the peak area thereof was calculated to be 154616 based on the absorption at a wavelength of 280 nm. The findings that elution position of the peak shifts more forward and that the peak area increased 2.6 times through adding arginine hydrochloride in place of sodium chloride suggested that unnecessary interaction between the human fibroblast growth factor and the column was attenuated and thereby the gel filtration chromatography was perfected more successfully. Additionally, in this instance, any peak of the aggregates was not detected regardless of addition or no addition of arginine, therefore, the content of the aggregates was not calculated.

### [Example 5]

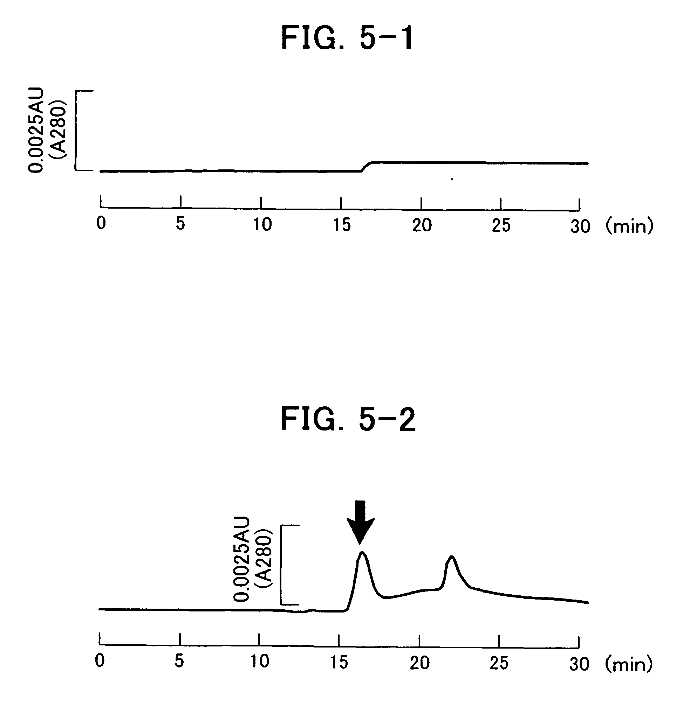

Five microliter of 0.96 mg/ml human interferon gamma (manufactured by Biosource Inc., Catalog No. PHC4031) dissolved in 40 mM Tris HCl buffer, pH 7.4 was subjected to gel filtration chromatography with a column of Superdex 75HR 10/30 (manufactured by Amersham Bioscience K.K.) using 0.1 M sodium phosphate, 0.4 M sodium chloride, pH 6.8 as the developing solvent at a flow rate of 0.8 ml/min, and the elution performance was studied using the ultraviolet absorption at a wavelength of 280 nm as a marker. As shown in Fig. 5-1, any elution peak could not been detected at all. Even though the sodium chloride concentration in the developing solvent was lowered to 0.2 M, and also to 0 M, any peak could not be detected similarly. Furthermore, even though 1 M urea that is a protein denaturating agent was added in place of sodium chloride, any peak could not be similarly detected. On the other hand, 5 µl of the completely same human interferon gamma was subjected to gel filtration chromatography with the completely same condition except that 0.4 M arginine hydrochloride was added in place of sodium chloride or urea in the developing solvent for the aforementioned gel filtration chromatography. Accordingly, as shown in 5-2, the elution peak could be found, and the peak area was calculated to be 26914 based on the absorption at a wavelength of 280 nm. As described hereinabove, gel filtration chromatography of human gamma interferon, the peak of which can be neither separated nor recovered by gel filtration chromatography in which a general buffer is used as the developing solvent, was enabled by merely adding arginine into the developing solvent. Additionally, in this instance, any peak of the aggregates was not detected regardless of addition or no addition of arginine, therefore, the content of the aggregates was not calculated.

### [Brief Description of the Drawings]

[Fig. 1-1] Gel filtration chromatography of mouse monoclonal antibody (0.2 M sodium chloride added to the developing solvent), wherein large arrowhead indicates the monomer, and small arrowhead indicates the aggregates. Details of the condition are set forth in the detailed description.
[Fig. 1-2] Gel filtration chromatography of mouse monoclonal antibody (0.2 M arginine hydrochloride added to the developing solvent), wherein large arrowhead indicates the monomer, and small arrowhead indicates the aggregates. Details of the condition are set forth in the detailed description.
[Fig. 2-1] Gel filtration chromatography of human activin (0.75 M sodium chloride added to the developing solvent), wherein large arrowhead indicates human activin. Solvent peak included in the sample overlapped with the part pointed by the arrowhead. Details of the condition are set forth in the detailed description.
[Fig. 2-2] Gel filtration chromatography of human activin (0.75 M arginine hydrochloride added to the developing solvent), wherein large arrowhead indicates the monomer, and small arrowhead indicates the aggregates. Peaks found later than 20 min are derived from the solvent included in the sample. Details of the condition are set forth in the detailed description.
[Fig. 3-1] Gel filtration chromatography of human interleukin 6 (0.75 M sodium chloride added to the developing solvent), wherein large arrowhead indicates the monomer, and small arrowhead indicates the aggregates. Peaks found later than 20 min are derived from the solvent included in the sample. Details of the condition are set forth in the detailed description.
[Fig. 3-2] Gel filtration chromatography of human interleukin 6 (0.75 M arginine hydrochloride added to the developing solvent), wherein large arrowhead indicates the monomer, and small arrowhead indicates the aggregates. Peaks found later than 20 min are derived from the solvent included in the sample. Details of the condition are set forth in the detailed description.
[Fig. 4-1] Gel filtration chromatography of human fibroblast growth factor (0.2 M sodium chloride added to the developing solvent), wherein arrowhead indicates the human fibroblast growth factor. Large peak found thereafter is derived from the solvent included in the sample. Details of the condition are set forth in the detailed description.
[Fig. 4-2] Gel filtration chromatography of human fibroblast growth factor (0.2 M arginine hydrochloride added to the developing solvent), wherein arrowhead indicates the human fibroblast growth factor. Large peak found thereafter is derived from the solvent included in the sample. Details of the condition are set forth in the detailed description.
[Fig. 5-1] Gel filtration chromatography of human interferon gamma (0.4 M sodium chloride added to the developing solvent), wherein details of the condition are set forth in the detailed description.
[Fig. 5-2] Gel filtration chromatography of human interferon gamma (0.4 M arginine hydrochloride added to the developing solvent), wherein arrowhead indicates the human interferon gamma. Peaks found later than 20 min are derived from the solvent included in the sample. Details of the condition are set forth in the detailed description.

### [Fig. 1-1]

Gel filtration chromatography of mouse monoclonal antibody (0.2 M sodium chloride added to the developing solvent): wherein large arrowhead indicates the monomer, and small arrowhead indicates the aggregates. Details of the condition are set forth in the detailed description.

### [Fig. 1-2]

Gel filtration chromatography of mouse monoclonal antibody (0.2 M arginine hydrochloride added to the developing solvent): wherein large arrowhead indicates the monomer, and small arrowhead indicates the aggregates. Details of the condition are set forth in the detailed description.

### [Fig. 2-1]

Gel filtration chromatography of human activin (0.75 M sodium chloride added to the developing solvent): wherein large arrowhead indicates human activin. Solvent peak included in the sample overlapped with the part pointed by the arrowhead. Details of the condition are set forth in the detailed description.

### [Fig. 2-2]

Gel filtration chromatography of human activin (0.75 M arginine hydrochloride added to the developing solvent): wherein large arrowhead indicates the monomer, and small arrowhead indicates the aggregates. Peaks found later than 20 min are derived from the solvent included in the sample. Details of the solvent condition are set forth in the detailed description.

### [Fig. 3-1]

Gel filtration chromatography of human interleukin 6 (0.75 M sodium chloride added to the developing solvent): wherein large arrowhead indicates the monomer, and small arrowhead indicates the aggregates. Peaks found later than 20 min are derived from the solvent included in the sample. Details of the condition are set forth in the detailed description.

### [Fig. 3-2]

Gel filtration chromatography of human interleukin 6 (0.75 M arginine hydrochloride added to the developing solvent): wherein large arrowhead indicates the monomer, and small arrowhead indicates the aggregates. Peaks found later than 20 min are derived from the solvent included in the sample. Details of the condition are set forth in the detailed description.

### [Fig. 4-1]

Gel filtration chromatography of human fibroblast growth factor (0.2 M sodium chloride added to the developing solvent): wherein arrowhead indicates the human fibroblast growth factor. Large peak found thereafter is derived from the solvent included in the sample. Details of the condition are set forth in the detailed description.

### [Fig. 4-2]

Gel filtration chromatography of human fibroblast growth factor (0.2 M arginine hydrochloride added to the developing solvent): wherein arrowhead indicates the human fibroblast growth factor. Large peak found thereafter is derived from the solvent included in the sample. Details of the condition are set forth in the detailed description.

### [Fig. 5-1]

Gel filtration chromatography of human interferon gamma (0.4 M sodium chloride added to the developing solvent): wherein details of the condition are set forth in the detailed description.

### [Fig. 5-2]

Gel filtration chromatography of human interferon gamma (0.4 M arginine hydrochloride added to the developing solvent): wherein arrowhead indicates the human interferon gamma. Peaks found later than 20 min are derived from the solvent included in the sample. Details of the condition are set forth in the detailed description.

## Claims

1. A method for improving recovery yield of an antibody, a hydrophobic protein or a hydrophobic peptide in a process of the purification with gel filtration chromatography of a solution containing at least one of antibodies including aggregates, hydrophobic proteins and hydrophobic peptides, the method comprising allowing 0.05 M to 1.5 M arginine and/or an arginine derivative to be contained in a developing solvent followed by development, wherein the arginine derivative is selected from the group consisting of acylated arginine, arginine butyl esters with the carboxyl group modified, agmatine with the carboxyl group eliminated and arginine having a hydroxyl group introduced in place of an α-amino group.

2. The method according to claim 1, wherein the concentration of the arginine and/or the arginine derivative in the developing solvent is 0.05 to 1.25 M.

3. The method according to claim 1 wherein the concentration of the arginine and/or the arginine derivative in the developing solvent is 0.10 to 0.75 M.

4. The use of an agent comprising arginine and/or an arginine derivative selected from the group consisting of acylated arginine, arginine butyl esters with the carboxyl group modified, agmatine with the carboxyl group eliminated and arginine having a hydroxyl group introduced in place of an α-amino group, for alleviating interaction between an antibody, a hydrophobic protein or a hydrophobic peptide and a column of gel filtration chromatography.

## Patentansprüche

1. Verfahren zum Verbessern der Ausbeute bei der Gewinnung eines Antikörpers, eines hydrophoben Proteins oder eines hydrophoben Peptids in einem Verfahren zur Reinigung einer Lösung, die mindestens ein Mitglied aus der aus Antikörpern, einschließlich Aggregaten, hydrophoben Proteinen und hydrophoben Peptiden bestehenden Gruppe enthält, durch Gelfiltrationschromatographie, wobei das Verfahren das Zulassen, das 0,05 M bis 1,5 M Arginin und/oder ein Argininderivat in einem Entwicklungslösungsmittel enthalten ist, und die nachfolgende Entwicklung umfasst, wobei das Argininderivat aus der Gruppe ausgewählt ist, die aus acyliertem Arginin, Argininbutylestern mit modifizierter Carboxygruppe, Agmatin mit eliminierter Carboxygruppe und Arginin mit einer anstelle einer α-Amingruppe eingeführten Hydroxygruppe besteht.

2. Verfahren nach Anspruch 1, wobei die Konzentration des Arginins und/oder Argininderivats in dem Entwicklungslösungsmittel 0,05 bis 1,25 M ist.

3. Verfahren nach Anspruch 1, wobei die Konzentration des Arginins und/oder Argininderivats in dem Entwicklungslösungsmittel 0,10 bis 0,75 M ist.

4. Verwendung eines Mittels, das Arginin und/oder ein Argininderivat, ausgewählt aus der Gruppe, die aus acyliertem Arginin, Argininbutylestern mit modifizierter Carboxygruppe, Agmatin mit eliminierter Carboxygruppe und Arginin mit einer anstelle einer α-Aminogruppe eingeführten Hydroxygruppe besteht, zur Verringerung der Wechselwirkung zwischen einem Antikörper, einem hydrophoben Protein oder einem hydrophoben Peptid und einer Gelfiltrationschromatographiesäule.

## Revendications

1. Procédé pour améliorer le rendement de récupération d'un anticorps, d'une protéine hydrophobe ou d'un peptide hydrophobe dans un procédé de purification avec une chromatographie de filtration sur gel d'une solution contenant au moins un des anticorps comprenant des agrégats, des protéines hydrophobes et des peptides hydrophobes, le procédé consistant à laisser une arginine et/ou un dérivé d'arginine 0,05 M à 1,5 M contenu dans un solvant de développement suivi par un développement, dans lequel le dérivé d'arginine est choisi parmi l'arginine acylée, des esters butyliques d'arginine avec le groupe carboxyle modifié, l'agmatine avec le groupe carboxyle éliminé et l'arginine présentant un groupe hydroxyle introduit à la place d'un groupe α-amino.

2. Procédé selon la revendication 1, dans lequel la concentration de l'arginine et/ou du dérivé d'arginine dans le solvant de développement est de 0,05 à 1,25 M.

3. Procédé selon la revendication 1, dans lequel la concentration de l'arginine et/ou du dérivé d'arginine dans le solvant de développement est de 0,10 à 0,75 M.

4. Utilisation d'un agent comprenant de l'arginine et/ou un dérivé d'arginine choisi parmi l'arginine acylée, des esters butyliques d'arginine avec le groupe carboxyle modifié, l'agmatine avec le groupe carboxyle éliminé et l'arginine présentant un groupe hydroxyle introduit à la place d'un groupe α-amino, pour alléger une interaction entre un anticorps, une protéine hydrophobe ou un peptide hydrophobe et une colonne de chromatographie de filtration sur gel.
